(19) Europäisches Patentamt
European Patent Office
Office européen des brevets

(11) Numéro de publication: **0 015 841**
**B1**

(12) **FASCICULE DE BREVET EUROPEEN**

(45) Date de publication du fascicule du brevet: **25.08.82**

(51) Int. Cl.³: **G 01 N 33/54, C 12 Q 1/34**

(21) Numéro de dépôt: **80400289.7**

(22) Date de dépôt: **29.02.80**

(54) **Nouveaux tests par agglutination pour la détection des virus de la grippe, réactifs pour la réalisation de ces tests, procédé de préparation de ces réactifs, application de ces réactifs et kit diagnostic contenant ces réactifs.**

(30) Priorité: **06.03.79 FR 7905703**

(43) Date de publication de la demande:
**17.09.80 Bulletin 80/19**

(45) Mention de la délivrance du brevet:
**25.08.82 Bulletin 82/34**

(84) Etats contractants désignés:
**AT BE CH DE GB IT LU NL SE**

(56) Documents cités:
**DE - A - 2 653 267**
**US - A - 3 826 613**

**JOURNAL OF BIOLOGICAL STANDARDIZATION, no. 4, 1976, pages 225—241 New York, U.S.A. O. THRAENHART et al.: "Standardization of a rapid modified microneuraminidase-inhibition test (Essen-NIT) for influenza virusneuraminidase antibody assay and comparison with the W.H.O. method".**

(73) Titulaire: **INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur: **Quash, Gérard Anthony, Dr.**
**63, Boulevard des Provinces**
**F-69110 Sainte Foy-Les Lyon (FR)**

(74) Mandataire: **Ores, Irène et al,**
**CABINET ORES 6, Avenue de Messine**
**F-75008 - Paris (FR)**

(56) Documents cités
**BIOCHIMICA ET BIOPHYSICA ACTA, no 523, 1978, pages 435442 Amsterdam, NL. U.V. SANTER et al.: "A rapid assay for neuraminidase. The detection of two differences in activity associated with virus transformation".**

EP 0 015 841 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Nouveaux tests par agglutination pour la détection des virus de la grippe, réactifs pour la réalisation de ces tests, procédé de préparation de ces réactifs, application de ces réactifs et kit diagnostic contenant ces réactifs.

La présente invention est relative à de nouveaux tests par agglutination pour la détection des virus de la grippe, et aux réactifs pour la réalisation de ces tests.

La grippe, maladie contagieuse, endémique et épidémique, dont pratiquement l'unique traitement est la vaccination, pose de graves problèmes prophylactiques étant donné la variabilité antigénique des virus de la grippe. D'une manière générale, la détection des virus des la grippe et le diagnostic résultant de la grippe sont importants pour trois raisons essentielles:

—sur le plan épidémioligique dans la surveillance du niveau immunitaire des populations lors d'une épidémie grippale;

—sur le plan préventif, pour essayer de prévoir les souches à introduire dans les vaccins:

—sur le plan clinique, enfin, pour le diagnostic précis de l'infection grippale, en particulier pour les formes cliniques anormales et rares.

C'est pourquoi des techniques ont été proposées depuis quelques années pour détecter les virus de la grippe. Ces techniques peuvent être classées dans deux catégories principales: les techniques biologiques et les techniques immunologiques, ces dernières comprenant les techniques classiques d'agglutination et les techniques d'inhibition de l'activité de la neuraminidase. La technique biologique qui consiste à isoler et à identifier le virus lui-même après culture sur embryon d'oeuf ou sur les cellules de rein de singe est longue et requiert un personnel hautement qualifié. Par contre, les techniques immunologiques sont d'une mise en oeuvre plus facile et sont donc plus largement utilisées que la technique bioligique, en particulier la méthode d'inhibition d'hémagglutination. L'hémagglutination n'est visible que dans le cas où sérum analysé ne contient pas les anticorps spécifiques qui masquent les sites hémagglutinants. Toutefois, ces techniques immunologiques présentent quelques difficultés parfois difficiles à surmonter, à savoir:

—la présence d'inhibiteurs non spécifiques,

—Les variations des caractéristiques biologiques et anti-géniques des virus,

—la variabilité de la qualité des érythrocytes.

D'autre part, l'inhibition de l'activité enzymatique de la neuraminidase (Mucopolysaccharide N-acétyl-neuraminylhydrolase des virus grippaux; classification internationale 3.2.1.18) constitue une très bonne méthode pour détecter les anticorps anti-neuraminidase spécifique d'une souche du virus grippal. La neuraminidase est identifiée par inhibition de l'activité de l'enzyme par un antisérum préparé contre les antigènes de virus de référence. La détection du virus de la grippe se résume en un dosage de la neuraminidase [cf. en particulier les travaux de P.CUATRECASAS et G. ILLIANO dans Biochem. and Biophys. Res. Communications (1971), *44* 1, 178—184]. Le dosage de la neuraminidase comporte deux étapes principales:

a) l'étape d'incubation du virus seul ou du virus prétraité par l'antisérum avec la fétuine—le substrat de la neuraminidase—pendant 18 heures à 37°C;

b) l'étape de dosage de l'acide sialique libéré par l'action de la neuraminidase à l'aide de la technique de WARREN [L. WARREN, J. Biol. Chem. *234* (1959) 1971]. Ce dernier dosage est très long et compliqué et comporte plusieurs étapes qui nécessitent des réactifs nombreux, des temps de chauffage précis et finalement des mesures colorimétriques après extraction du dérivé thiobarbiturique de l'acide sialique oxydé dans du BuOH/HCl. Le dosage en routine des plusieurs échantillons est donc particulièrement laborieux (cf. également concernant le dosage de l'acide sialique en vue de la détection de la neuraminidase, les Articles suivants: Journal of Biological Standardization 1976 4, 225—241, O. THRAENHART et E. K. KUWERT et Biochimica et Biophysica Acta, 523 (1978) 435—442, U. V. SANTER, J. YEE-FOON et M. C. GLICK).

La présente invention s'est en conséquence fixé pour but de pourvoir à un nouveau test de détection des virus de la grippe qui allie la spécificité de la réaction enzymatique aux techniques d'agglutination, qui est facile à réaliser et qui supprime les inconvénients de ces deux méthodes prises séparément.

La présente invention a pour objet un nouveau procédé de détection des virus de la grippe, caractérisé en ce qu'on réalise un test d'agglutination par:

— mise en contact de virus de la grippe ou de liquide allantoïque contenant ledit virus, avec un substrat de la neuraminidase comportant au moins un groupement acide sialique, lequel substrat est fixé par couplage par covalence sur un support solide insoluble approprié, étant entendu que ledit substrat peut être constitué par tous types de mucoprotéines, mucopolysaccharides, oligosaccharides, ou mucolipides comportant au moins un groupement acide sialique apte à jouer le rôle de substrat de la neuraminidase;—incubation du virus de la grippe avec son substrat pendant un temps approprié;—puis lecture de la réactions.

Selon un mode de réalisation avantageux du test de détection de virus de la grippe conforme à l'invention, les dilutions voulues de virus de la grippe ou de liquide allantoïque et le substrat de neuraminidase couplé par covalence audit support solide, sont mis en suspension dans un tampon

approprié, puis on réalise, préalablement à la lecture de la réaction, l'incubation pendant la durée voulue.

Selon un autre mode de réalisation avantageux du test de détection de virus de la grippe conforme à l'invention, le milieu réactionnel comprenant les dilutions voulues de virus de la grippe ou de liquide allantoïque, le substrat de neuraminidase couplé par covalence à un support solide et le tampon, est introduit dans les alvéoles diune microplaque d'agglutination connue en ellemême puis incubé après quoi la lecture de la réaction est effectuée directement dans la plaque d'agglutination, à l'oeil nu, à l'aide d'une source de lumière diffuse.

Selon un mode de réalisation particulièrement avantageux de l'objet de l'invention, la substrat de neuraminidase fixé sur le support est de la fétuine.

Conformément à l'invention, le support solide insoluble est pris notamment dans le groupe qui comprend les sphères de latex, les billes de gel d'agarose ou de dextrâne, les billes de verre activées, ou analogues.

Selon un mode de réalisation avantageux dudit support solide, ce dernier porte des chaînes latérales comportant un groupement $—NH_2$ terminal.

Suivant un mode de réalisation avantageux de l'objet de l'invention, le support solide comporte des groupements carboxyles sur lesquels sont fixées par covalence, lesdîtes chaînes latérales portant un groupement $—NH_2$ terminal.

Suivant un autre mode de réalisation avantageux de l'objet de l'invention, le support solide est constitué par des sphères latex acide-hydrazide de formule I ciaprès:

$$Latex—CO—NH—(CH_2)_6—NH—CO—(CH_2)_2—CO—NH—NH_2 \qquad (I)$$

Selon un autre mode de réalisation avantageux de l'objet de l'invention, le support solide est constitué par de sphères de latex portant des chaînes latérales p-aminobenzoate dont la diazotation donne lieu à des liaisons avec des résidus tyrosine de glycoprotéines contenant des résidus acide sialique dans leurs fractions glycosidiques.

La préparation de ces différents supports a été décrite dans les Brevets et Certificats d'Addition français n° 75 34627; 76 08966; 76 25898 et 77 01889.

Conformément à l'invention, on peut préparer également ces supports par un procédé comportant les étapes suivantes:

a) traitement du latex-acide par l'hexaméthylènediamine (HMD) dissoute extemporanément en présence de 1-éthyl-3-3-diméthylaminopropylcarbodiimide (EDC) ajouté à l'état solide en trois additions en l'espace de 24 heures;

b) succinylation des groupements $—NH_2$ par addition d'anhydride succinique à un pH supérieur à 7;

c) traitement au chlorhydrate d'hydrazine en présence d'EDC.

Ainsi, le procédé de détection des virus de la grippe conforme à l'invention ne détecte pas la neuraminidase par dosage de l'acide sialique produit lors de la réaction enzymatique, mais consiste à mesurer le complexe formé entre l'enzyme et son substrat, à savoir la fétuine, immobilisés sur des sphères de latex, ce qui donne naissance à une agglutination des sphères.

Schématiquement, la réaction est la suivante:

$$E + S \rightarrow ES \rightarrow E + P$$

où E = enzyme; (neuraminidase de la grippe) S = substrat; (fétuine) P = produit (acide sialique)

ES = complexe

On obtient ainsi la même spécificité qu'avec la réaction enzymatique, mais avec l'avantage important d'éliminer les étapes ultérieures qui sont nécessaires, con-formément à l'Art antérieur, pour la mise en évidence de l'acide sialique libéré.

La présente invention a également pour objet un réactif pour la mise en oeuvre du test d'agglutination conforme à l'invention, constitué par un substrat de neuraminidase comportant au moins un groupement acide sialique, fixé par couplage par covalence sur un support solide insoluble approprié.

Conformément à l'invention, ledit substrat est choisi dans le groupe qui comprend les mucoprotéines, les mucopolysaccharides, les oligosaccharides et les mucolipides comportant au moins un groupement acide sialique.

Egalement conformément à l'invention, ledit substrat est couplé par covalence sur un support solide insoluble du type précité, préalablement activé par diazotation à l'aide de $NaNO_2$ en solution acide.

Pour la préparation du réactif conforme à l'invention, on procède au couplage du substrat de la neuraminidase sur le support solide insoluble, dans un tampon approprié, après avoir éventuellement

fixé sur ledit support des chaînes latérales comportant un groupement —$NH_2$ terminal et après avoir éventuellement activé ledit support par diazotation à l'aide de $NaNO_2$ en solution acide.

Outre les dispositions qui précèdent, l'invention comprend encore d'autres dispositions, qui ressortiront de la description qui va suivre.

La présente invention vise plus particulièrement la détection des virus de la grippe par un procédé simple, rapide, reproductible, très sensible et d'un coût relativement peu élevé, les moyens propres à la mise en oeuvre de ce procédé et les procédés d'ensemble dans lesquels sont inclus le procédé et les réactifs conformes à la présente invention.

L'invention sera mieux comprise à l'aide du complément de description qui va suivre et qui se réfère à un exemple de mise en oeuvre du test conforme à la présente invention.

Il doit être bien entendu, toutefois, que cet exemple est donné uniquement à titre d'illustration de l'objet de l'invention, mais n'en constitue en aucune manière une limitation.

## EXEMPLE

1—*Préparation du support solide: latex-acide-hydrazide*

On couple à la surface de sphères de latex carboxylées des chaînes d'hydrazide.

La synthèse a lieu selon le schéma suivant:

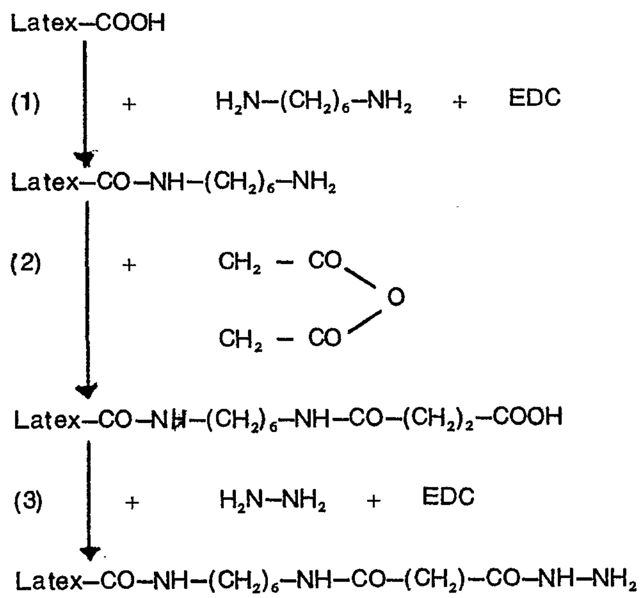


1 g de sphères de latex K318C (fabriquées par RHONE-POULENC, Aubervilliers — France) de 0,85 $\mu$m de diamètre et contenant 5,5 . $10^{15}$ COOH/mg, lavé par filtration sur filtre Millipore (diamètre 450 mm, porosité 0,6 $\mu$m) est repris par 2 ml de tampon Borate HCl 0,1M pH 8,1 dans un tube de 30 ml centrifugeable.

On y ajoute 230 $\mu$moles d'hexaméthylènediamine (HMD) dissoute extemporanément dans 25 ml de tampon BBS (Borate HCl 0,01M pH 8,1 0,15 M NaCl).

Le tube est placé sur agitateur oscillant à 4°C et reçoit en trois additions espacées sur 24 heures, de 1-éthyl-3-3-diméthylaminopropylcarbodiimide (EDC) à l'état solide, la concentration finale étant 0,05M EDC.

Les lavages sont effectués en trois cycles de centrifugation de 15 minutes chacun à 12 000 g en tampon BBS.

Le culot obtenu est repris par du tampon Borate HCl 0,1M pH 8,1 et les groupements —$NH_2$ sont succinylés par l'addition de 1 mmole d'anhydride succinique solide. Après une nuit de contact, le tube est centrifugé, le culot repris de nouveau par du tampon Borate et le traitement est renouvelé.

Après trois traitements, les latex sont lavés par trois cycles de centrifugation. La succinylation doit se faire à un pH supérieur à 7, que l'on ajuste si nécessaire, avec NaOH 1N. C'est pour cela que dans cette étape, le tampon Borate remplace le BBS.

Le culot de latex obtenu est repris par 2 ml de Borate 0,1M pH 8,1; 2 ml d'une solution d'hydrazine-HCl pH 7,5, 0,2M sont alors ajoutés. La suspension est soigneusement homogénéisée et le volume est porté à 25 ml par du BBS pH 8,1, et l'on peut alors ajouter l'EDC 0,05M final. La préparation est incubée une nuit à +4°C sur agitateur oscillant. Le traitement à l'hydrazine EDC est renouvelé.

Le cycle de trois lavages est suivi d'une dialyse prolongée. On obtient ainsi 3,6 . $10^{15}$ hydrazide/mg de latex titrés par diazotation, soit un rendement de 66%.

2—*Couplage du latex-acide-hydrazide à la fétuine*

a) Activation du latex-acide-hydrazide

A 200 mg de latex hydrazide en suspension dans l'eau, on ajoute 4 ml de $NaNO_2$ 4% et 10 ml d'HCl 2N. Le mélange est maintenu sous agitation dans un bain de glace pendant 15 minutes. A la fin de cette période, le mélange est rapidement filtré sur un filtre Millipore 0,65 $\mu$m et lavé avec un tampon glacé contenant 0,1M Borate-HCl pH 8,1 pour éliminer l'excès d'$HNO_2$, $NaNO_2$ et HCl.

Le latex diazoté est décroché du Millipore par agitation sur vortex et traitement aux ultra-sons.

b) Couplage proprement dit

A 100 mg de latex activé, on ajoute 100 mg de fétuine dissoute au tampon borate HCl 0,1M pH 8,1 dans un volume total de 20 ml. Le mélange est laissé en contact sous agitation douce pendant 48 heures à 4°C. A la fin de cette période, le latex-fétuine est récupéré par centrifugation à 10 000 g pendant 20 minutes, et lavé par deux cycles successifs de centrifugation dans un tampon contenant 0,14M NaCl, 0,1M glycine-NaOH pH 8,1. A la fin des lavages, le latex est repris dans ce dernier tampon et resuspendu à l'aide d'un traitement aux ultra-sons.

Le dosage de protéines restantes dans le surnageant par rapport à la quantité mise, permet d'évaluer la quantité de fétuine fixée/mg de latex pour quatre préparations différentes de latex-fétuine, à 162 $\pm$ 35 $\mu$g.

3—*Test d'agglutination sur microplaque de latex-fétuine*

Tampon: 0,14M NaCL, 0,2% SAB (sérum albumine bovine),
0,05% Triton X 100 0,1M glycine-NaOH pH 8,1.

Des microplaques de 96 trous à alvéoles en "V" sont siliconées avant l'utilisation pour diminuer les phénomènes dûs à la tension superficielle.

On met dans chaque trou, dans l'ordre suivant:

a) 50 $\mu$l du tampon ci-dessus,

b) 50 $\mu$l des différentes dilutions de virus de la grippe ou de liquide allantoïque dilué dans 0,14M NaCl,

c) 50 $\mu$l de latex-fétuine dilué à 350 $\mu$g de latex/ml dans 0,14M NaCl, 0,1M glycine pH 8,1.

Un premier témoin de latex seul est fait avec 50 $\mu$l de 0,14M NaCl; un deuxième témoin contient la même quantité de latex, mais en présence de diverses dilutions de liquide allantoïque ne contenant pas de virus de la grippe.

On ferme la plaque avec un couvercle, on entoure de sparadrap et on place sur un agitateur de plaques à l'étuve à 37°C pendant 1 heure, suivie par un temps d'incubation d'une nuit de repos et à la température du laboratoire.

Lecture de la plaque:

On place les plaques sur un négatoscope et on lit contre un fond noir.

Elle peut se faire de deux façons:

Première méthode:

— réaction négative (symbolisée par — dans le Tableau I ci-après), il n'y a pas d'agglutination: présence d'un culot de billes formant un point net bien délimité

— réaction positive (symbolisée par +), il y a agglutination, c'est-à-dire dépôt des billes sous forme d'un tapis diffus mal délimité.

Deuxième méthode:

Elle se fait après inclinaison de la plaque pendant 15 à 20 minutes.

— Réaction négative: les billes non agglutinées se mettent à couler et font une traînée blanchâtre

— réaction positive: les billes agglutinées ne coulent pas.

Donc, pour qu'un test soit interprétable, il faudra que tous le témoins, à savoir le témoin latex en présence de tampon et le témoin latex en présence de liquide allantoïque seul, soient négatifs, c'est-à-dire qu'ils devront donner un culot de billes qui, après inclinaison, coulera.

TABLEAU I

| | Dilutions | | | | | | | | |
|---|---|---|---|---|---|---|---|---|---|
| | 1/10 | 1/20 | 1/40 | 1/80 | 1/160 | 1/320 | 1/640 | T | T |
| Liquid allantoïque contenant le virus de la grippe A | + | + | + | + | + | + | – | – | – |
| Liquid allantoïque témoin | – | – | – | – | – | – | – | – | – |

La réaction entre la grippe A et le latex-fétuine est donc spécifique.

Pour mettre en évidence la sensibilité du test de détection de virus de la grippe conforme à l'invention, on a comparé celui-ci aux tests actuellement utilisés. Les résultats des tests comparatifs réalisés sont réunis dans le Tableau II ci-aprés

TABLEAU II

TESTS COMPARATIFS POUR DIFFERENTES SOUCHES DE GRIPPE ENTRE LES TITRES OBTENUS PAR DOSAGE ENZYMATIQUE ET PAR AGGLUTINATION

| | TITRES | | |
|---|---|---|---|
| | | NEURAMINIDASE | |
| | HEMAGGLUTI-NINE classique | par dosage enzymatique | par agglutination latex |
| CULTURES SUR OEUF | | | |
| Vict 3/75 (9.9.77) 79 all | 5120 | 10 | 320 |
| Texas 1/77 (6.3.78) 35 all | 5120 | 15 | 160 |
| URSS 90/77 (6.3.78) 30 + 31 all | 40 | 20 | 90 |
| MRC 2 (14.4.78) mell all | >10240 | 20 | 320 |
| England 42/72 (29.3.78) mell all | 1280 | 10 | 160 |
| England HCO 658 (17.3.78) 2 all | >10240 | 20 | 320 |
| England HOO 743 (3,4.78) 5 all | >10240 | 15 | 160 |
| England HOO 746 (14,4.78) 12 all | 320 | 60 | 160 |
| CULTURES SUR CELLULES | | | |
| England p2 RSV 658 (27.2.78) | 5 | 0 | 80 |
| England p4 RSV 743 (13.3.78) | 5 | 0 | 40 |
| England p4 RSV 746 (23.3.78) | 10 | 0 | 10 |

Il se dégage nettement de ce Tableau comparatif que le procédé conforme à l'invention est très sensible comparativement au procédé de dosage classique de neuraminidase par la méthode de WARREN.

En effet, alors qu'il est pratiquement impossible de déceler l'activité neuraminidasique des cultures sur cellules par voie enzymatique, la méthode latex-fétuine décèle la neuraminidase.

Le Tableau III ci-après reproduit les résultats d'une série d'essais effectués en vue de mettre en évidence la spécificité des anticorps anti-neuraminidase sur l'agglutination des réactifs sphères de latex-fétuine par les virus de la grippe.

Ces essais ont été effectués en microplaque, le virus de la grippe testé étant un virus de grippe SINGAPOUR 57 dilué au 1/50 dans NaCl 0,14M.

Les antisérums testés ont été dilués dans NaCl 0,14M. Les essais ont été réalisés comme suit:

— on place dans chaque alvéole:

25 $\mu$l de la dilution d'antisérum à tester

+25 $\mu$l de Singapour 1/50

— on incube à 22°C sous agitation rotative, pendant 1 heure.

— Dans chaque alvéole, on ajoute ensuite:

50 $\mu$l d'un tampon présentant la composition suivante:

| | |
|---|---|
| NaCl | 0,14M |
| Gly-NaOH | 0,10M, pH 8,1 |
| SAB | 0,20% |
| Triton X 100 | 0,05% |

+50 $\mu$l de sphères de latex-fétuine, diluées à 350 $\mu$g latex-ml

— on agite pendant 1 heure à 37°C, puis on laisse reposer une nuit à 22°C.

— Puis on procède à la lecture de la plaque comme décrit à l'Exemple ci-dessus.

TABLEAU III

Tests de spécificité des anticorps anti-neuraminidase par inhibition d'agglutination

| Antisérums anti | Origine | DILUTIONS | | | | | | | | TEMOINS | | | |
|---|---|---|---|---|---|---|---|---|---|---|---|---|---|
| | | 1/160 | 1/320 | 1/640 | 1/1280 | 1/2560 | 1/5120 | 1/10240 | 1/20480 | Liquide allantoïque seul 1/50 | | Liquide allantoïque 1/50 + Singapour 57 | |
| Grippe Texas 77 | Furet | + | + | + | + | + | + | + | + | – | – | + | + |
| Neuraminidase Texas 77 | Lapin | – | + | + | + | + | + | + | + | – | – | + | + |
| Grippe Singapour 57 | Furet | – | – | – | – | + | + | + | + | | | | |
| Neuraminidase Singapour 57 | Lapin | – | – | – | – | – | – | + | + | | | | |
| Grippe URSS 77 | Lapin | – | + | + | + | + | + | + | + | | | | |
| Grippe SM 1 | Furet | + | + | + | + | + | + | + | + | | | | |
| Grippe SM 1 | Lapin | – | + | + | + | + | + | + | + | | | | |

Il ressort clairement de ce Tableau que l'inhibition de l'agglutination n'a lieu qu'avec les sérums homologues anti-Singapour 57.

Il ressort de la description qui précède que quels que soient les modes de mise en oeuvre, de réalisation et d'application adoptés, l'on obtient un procédé de détection des virus de la grippe et, par voie de conséquence un test de diagnostic de la grippe, qui présentent par rapport aux procédés visant au même but, antérieurement connus, (biologique, immunologique ou enzymatique), des avantages très importants, outre ceux qui ont déjà été mentionnés dans ce qui précède, et notamment:

A—*La rapidité*

Cette méthode est très facile à réaliser.

Elle est rapide, se réalise facilement en une demi-journée; le lendemain, la lecture ne demande aucune manipulation à la différence du titrage enzymatique de la neuraminidase, qui est long.

Les réactifs, c'est-à-dire la solution de billes et le tampon, se conservent très bien à +4°C. Il n'est donc pas nécessaire de les repréparer à chaque manipulation, contrairement au dosage enzymatique de la neuraminidase qui nécessite une préparation extemporanée des réactifs.

Elle nécessite une surveillance moins constante, une fois les dilutions effectuées et, la plaque remplie, la manipulation est pratiquement finie.

B—*La reproductibilité*

Trois souches virales testées simultanément ont été reprises deux fois de suite, ces trois souches étant:

— Grippe A Vict 3/75 (9.9.77) 79 all
— Grippe A URSS 90/77 (6.3.78) 30 + 31 all
— Grippe A Texas 1/77 (6.3.78) 35 all

Les tests ont donné les résultats suivants:

Test 1:

| | | |
|---|---|---|
| | Vict | 320 |
| | URSS | 80 |
| | Texas | 160 |

Test 2:

| | | |
|---|---|---|
| | Vict | 320 |
| | URSS | 80 |
| | Texas | 160 |

Il y a donc reproductibilité des résultats. (Les souches ont été congelées à environ −20°C entre les deux dosges).

C—*La Sensibilité*

La sensibilité de la méthode a été prouvée en déterminant la dilution de virus jusqu'à laquelle la fétuine est capable d'être reconnue par son enzyme, la neuraminidase, et en comparant cette dilution au titre de neuraminidase obtenu par dosage enzymatique de la méthode de WARREN.

On a donc fait parallèlement pour différentes souches, les titrages d'agglutination sur latex-fétuine, les titrages de neuraminidase par la méthode de WARREN, ainsi que le titrage d'hémagglutinine par la méthode courante avec les globules rouges de poule, ces souches provenant de cultures sur oeuf ou sur cellules de rein de singe. Les résultats obtenus, récapitulés dans le Tableau II ci-dessus, font apparaître clairement la sensibilité du test conforme à l'invention.

D—*Economie*

1) Système de lecture:

La lecture ne demande aucun appareillage sophistiqué; elle est faite directement dans les plaques d'agglutination, à l'oeil nu, à l'aide d'une lumière rasante, sur fond noir, fournie par un négatoscope par exemple.

2) Stabilité des préparations:

Les préparations de latex-fétuine peuvent être conservées dans un tampon glycine, NaCl + 0,01% d'azide de Na à +4°C pendant plus de 6 mois.

E—*Autres applications*

Le réactif latex-fétuine peut également être utilisé pour le dosage des anticorps anti-neuraminidase dans le sérum humain.

Ainsi que cela ressort de ce qui précède, l'invention ne se limite nullement à ceux de ses modes de mise en oeuvre, de réalisation et d'application qui viennent d'être décrits de façon plus explicite; elle en embrasse au contraire toutes les variantes qui peuvent venir à l'esprit du technicien en la matière, sans

s'écarter du cadre, ni de la portée, de la présente invention.

**Revendications**

1. Procédé de détection des virus de la grippe, caractérisé en ce qu'on réalise un test d'agglutination par:— mise en contact de virus de la grippe ou de liquide allantoïque contant ledit virus avec un substrat de la neuraminidase comportant au moins un groupement acide sialique, lequel substrat est fixé par couplage par covalence sur un support solide insoluble approprié, étant entendu que ledit substrat peut être constitué par tous types de mucoprotéines, mucopolysaccharides, oligosaccharides, ou mucolipides comportant au moins un groupement acide sialique apte à jouer le rôle de substrat de la neuraminidase;—incubation du virus de la grippe avec son substrat pendant un temps approprié;—puis lecture de la réaction.

2. Procédé selon la Revendication 1, caractérisé en ce que les dilutions voulues de virus de la grippe ou de liquide allantoïque est le substrat de neuraminidase couplé par covalence audit support solide, sont mis en suspension dans un tampon approprié, puis on réalise, préalablement à la lecture de la réaction, l'incubation pendant la durée voulue.

3. Procédé selon l'une quelconque des Revendications 1 à 2, caractérisé en ce que le milieu réactionnel comprenant les dilutions voulues de virus de la grippe ou de liquide allantoïque, le substrat de neuraminidase couplé par covalence à un support solide et le tampon, est introduit dans les alvéoles d'une microplaque d'agglutination connue en elle-même, puis uncubé, après quoi la lecture de la réaction est effectuée directement dans la plaque d'agglutination, à l'oeil nu, à l'aide d'une source de lumière diffuse.

4. Procédé selon l'une quelconque des Revendications 1 à 3, caractérisé en ce que substrat de neuraminidase fixé sur le support est de la fétuine.

5. Procédé selon l'une quelconque des Revendications 1 à 4, caractérisé en ce que ledit support solide insoluble est pris notamment dans le groupe qui comprend les sphères de latex, les billes de gel d'agarose ou de dextrane, les billes de verre activées ou analogues.

6. Procédé selon la Revendication 5, caractérisé en ce que ledit suport solide porte des chaînes latérales comportant un groupement —$NH_2$ terminal.

7. Procédé selon l'une quelconque des Revendications 1 à 6, caractérisé en ce que ledit support solide comporte des groupements carboxyles sur lesquels sont fixées par covalence lesdites chaînes latérales portant un groupement —$NH_2$ terminal.

8. Procédé selon la Revendication 7, caractérisé en ce que le support solide est constitué par des sphères de latex acide-hydrazide de formule I ci-après:

$$\text{Latex—CO—NH—}(CH_2)_6\text{—NH—CO—}(CH_2)_2\text{—CO—NH—}NH_2 \qquad \text{(I)}$$

9. Procédé selon la Revendication 7, caractérisé en ce que le support solide est constitué par des sphères de latex portant des chaînes latérales p-aminobenzoate dont la diazotation donne lieu à des liaisons avec des résidus tyrosine de glycoprotéines contenant des résidus acide sialique dans leurs fractions glycosidiques.

10. Réactif pour la mise en oeuvre du procédé selon l'une quelconque des Revendications 1 à 9, caractérisé en ce qu'il est constitué par un substrat de neuraminidase comportant au moins un groupement acide sialique, fixé par couplage par covalence sur un suport solide insoluble approprié.

11. Réactif selon la Revendication 10, caractérisé en ce qu'il est choisi dans le groupe qui comprend les mucoprotéines, les mucopolysaccharides, les oligosaccharides et les mucolipides comportant au moins un groupement acide sialique.

12. Réactif selon l'une quelconque des Revendications 10 et 11, caractérisé en ce que ledit substrat est couplé par covalence sur un support solide préalablement activé par diazotation à l'aide de $NaNO_2$ en solution acide.

13. Réactif selon l'une quelconque des Revendications 10 à 12, caractérisé en ce qu'il contient comme agents de conservation, un tampon glycine, NaCl et 0,01% d'azide de Na, à + 4°C.

14. Procédé de préparation du réactif pour la mise en oeuvre du procédé selon l'une quelconque des Revendications 7 et 8, caractérisé ce qu'il comprend les étapes suivantes:

a) traitement de sphères de latex-acide par l'hexaméthylènediamine (HMD) dissoute extemporanément en présence de 1-éthyl-3-3-diméthylaminopropylcarbodiimide (EDC) ajouté à l'état solide en trois additions en l'espace de 24 heures,

b) succinylation des groupements —$NH_2$ par addition d'anhydride succinique à un pH supérieur à 7,

c) traitement au chlorhydrate d'hydrazine en présence d'EDC,

d) couplage par covalence du substrat de la neuraminidase comportant au moins un groupement acide sialique, de préférence en fin de chaîne, sur les sphères de latex acide-hydrazide obtenues à l'étape précédente, dans un tampon approprié.

15. Procédé selon la Revendication 14, caractérisé en ce que le couplage par covalence dudit substrat sur les sphères de latex axide-hydrazide est précédé d'une activation desdites sphères par

diazotation à l'aide de $NaNO_2$ en solution acide.

16. Application du réactif selon l'une quelconque des Revendications 10 à 13 au dosage d'anticorps antineuraminidase dans le sérum.

17. Kit de diagnostic caractérisé en ce qu'il contient comme réactif, un réactif selon l'une quelconque des Revendications 10 à 13 ou un réactif préparé par le procédé selon la Revendication 14.

**Claims**

1. Method for the detection of influenza virus, characterised in that an agglutination test is carried out by:—contacting the influenza virus or allantoic fluid containing said virus, with a neuraminidase substrate including at least one sialic acid group, which substrate is fixed by covalent bonding to a suitable insoluble solid support, it being understood that said substrate may be constituted by any type of mucoprotein, mucopolysaccharide, oligosaccharide or mucolipid including at least one sialic acid group adapted to act as a neuraminidase substrate:—incubation of the influenza virus with its substrate for a suitable time;—the reading the reaction.

2. Method according to claim 1, wherein the desired dilutions of influenza virus or of allantoic fluid and the neuraminidase substrate bonded by covalency to said solid support, are suspended in a suitable buffer, and then, prior to reading the reaction, incubation for the desired time is carried out.

3. Method according to any one of claims 1 or 2, wherein the reaction medium comprising the desired dilutions of influenza virus or allantoic fluid, the neuraminidase substrate bonded by covalency to a solid support and the buffer, is introduced into the cups of an agglutination microplate known in itself, then incubated, after which the reading of the reaction is carried out directly in the agglutination plate, by naked eye, by means of a diffused light source.

4. Method according to any one of claims 1 to 3, characterised in that the neuraminidase substrate fixed to the support is fetuin.

5. Method according to any one of claims 1 to 4, wherein said insoluble solid support is selected particularly from the group which comprises latex spheres, beads of agarose gel or dextran, activated glass beads or the like.

6. Method according to claim 5, wherein said solid support bears side chains including a terminal $—NH_2$ group.

7. Method according to any one of claims 1 to 6, wherein said solid support comprises carboxyl groups on which are fixed by covalency said side chains bearing a terminal $—NH_2$ groups.

8. Method according to claim 7, wherein the solid support is constituted by spheres of acid latex-hydrazide of following formula I:

$$\text{Latex—CO—NH—(CH}_2)_6\text{—NH—CO—(CH}_2)_2\text{—CO—NH—NH}_2 \qquad \text{(I)}$$

9. Method according to claim 7, wherein the solid support is constituted by latex spheres bearing p-aminobenzoate side chains whose diazotization gives rise to bonds with tyrosine residues of glycoproteins containing sialic acid residues in their glycoside fractions.

10. Reagent for practising the method according to any one of claims 1 to 9, characterised in that it is constituted by a neuraminidase substrate including at least one sialic acid group, fixed by covalent bonding to a suitable insoluble solid support.

11. Reagent according to claim 10, characterised in that it is selected from the group which comprises mucoproteins, mucopolysaccharides, oligosaccharides and mucolipids including at least one sialic acid group.

12. Reagent according to any one of claims 10 and 11, wherein said substrate is bonded by covalency to a solid support previously activated by diazotization by means of $NaNO_2$ in acid solution.

13. Method according to any one of claims 10 to 12, characterised in that it contains as preserving agents, a glycine buffer, NaCl and 0.01% of Na azide, at +4°C.

14. Method of preparing a reagent for practising the method according to any one of claims 7 and 8, characterised in that it comprises the following steps:

a) treating acid-latex spheres with hexamethylenediamine (HMD) dissolved extemporaneously in the presence of 1-ethyl-3-3-dimethylaminoproplycarbodiimide (EDC) added in the solid state in three additions in the space of 24 hours,

b) succinylation of the $—NH_2$ groups by the addition of succinic anhdride at a pH higher than 7,

c) treatment with hydrazine hydrochloride in the presence of EDC,

d) covalent bonding of the substrate of the neuraminidase including at least one sialic acid group, preferably at the chain end, on spheres of acid latex-hydrazide obtained in the preceding step, in a suitable buffer.

15. Method according to claim 14, wherein the covalent bonding of said substrate on the spheres of acid latex-hydrazide is preceded by activation of said spheres by diazotization by means of $NaNO_2$ in acid solution.

16. Application of the reagent according to any one of claims 10 to 13 to the determination of antineuraminidase antibodies in serum.

17. Diagnostic kit characterised in that it contains as reagent, a reagent according to any one of claims 10 to 13 or a reagent prepared by the method according to claim 14.

**Patentansprüche:**

1. Verfahren zum Nachweis von Grippeviren, dadurch gekennzeichnet, daß man einen Agglutinationstest durchführt, indem man:—Grippevirus oder genannten Virus enthaltende allantoische Flüssigkeit in Kontakt bringt mit einem Substrat der Neuraminidase, das wenigstens eine Sialinsäuregruppe enthält, wobei das Substrat durch kovalente Kupplung auf einem entsprechenden festen, unlöslichen Träger fixiert ist, wobei genannantes Substrat aus allen Arten von Mucoproteinen, Mucopolysacchariden, Oligosacchariden oder Mucolipiden bestehen kann, die wenigstens eine Sialinsäuregruppe enthalten, welche geeignet ist, die Rolle des Substrats der Neuraminidase zu spielen;—Inkubation des Grippevirus mit seinem Substrat während angemessener Zeit;—dann Ablesen der Reaktion.

2. Verfahren gemäß Anspruch 1, dadurch gekennzeichnet, daß die gewünschte Verdünnung des Grippevirus oder der allantoischen Flüssigkeit und das kovalente an genannten festen Träger gekuppelte Substrat der Neuraminidase in einem entsprechenden Puffer in Suspension gebracht werden, dann führt man vor dem Ablesen der Reaktion die Inkubation für die gewünschte Dauer durch.

3. Verfahren gemäß Ansprüchen 1 bis 2, dadurch gekennzeichnet, daß das Reaktionsmedium, welches die gewünschten Verdünnungen des Grippevirus oder der allantoischen Flüssigkeit, das kovalent auf einen festen Träger gekuppelte Substrat der Neuraminidase und den Puffer enthält, in die Zellen einer an sich bekannten Mikroagglutinationsplatte eingeführt wird, danach inkubiert wird, wonach das Ablesen der Reaktion direkt in der Agglutinationsplatte mit bloßem Auge mit Hilfe einer diffusen Licht-quelle durchgeführt wird.

4. Verfahren gemäß einem der Ansprüche 1 bis 3, dadurch gekennzeichnet, daß das auf dem Träger fixierte Neuraminidase- Substrat aus fötalem Protein besteht.

5. Verfahren gemäß einem der Ansprüche 1 bis 4, dadurch gekennzeichnet, daß der genannte feste unlösliche Träger insbesondere aus der Gruppe genommen ist, die Latexkugeln, Kugeln von Agarose-Gel oder Dextran, Kugeln aus aktiviertem Glas oder Ähnliches umfasst.

6. Verfahren gemäß Anspruch 5, dadurch gekennzeichnet, daß der genannte feste Träger Seitenketten trägt, die eine endständige $NH_2$-Gruppe enthalten.

7. Verfahren gemäß einem der Ansprüche 1 bis 6, dadurch gekennzeichnet, daß der genannte feste Träger Carboxylgruppen enthält, auf welchen die genannten Seitenketten kovalent fixiert sind, die eine endständige $NH_2$Gruppe tragen.

8. Verfahren gemäß Anspruch 7, dadurch gekennzeichnet, daß der feste Träger aus Säurehydrazid-Latexkugeln der Formel I besteht:

$$Latex—CO—NH—(CH_2)_6—NH—CO—(CH_2)_2—CO—NH—NH_2 \qquad (I)$$

9. Verfahren gemäß Anspruch 7, dadurch gekennzeichnet, daß der feste Träger aus Latexkugeln besteht, die p-Aminobenzoat-Seitenketten tragen, deren Diazotierung Verknüpfungen mit Tyrosin-Resten von Glykoproteinen, die Sialinsäurereste in ihren glykosidischen Anteilen enthalten ergibt.

10. Reagenz zur Durchführuhg des Verfahrens gemäß einem der Ansprüche 1 bis 9, dadurch gekennzeichnet, daß es aus einem Neuraminidase-Substrat besteht, das mindestens eine Sialinsäure-Gruppe enthält, die durch kovalente Kupplung auf einem entsprechenden festen unlöslichen Träger fixiert ist.

11. Reagenz nach Anspruch 10, dadurch gekennzeichnet, daß es gewählt ist aud der Gruppe, die die Mucoproteine, die Mucopolysaccharide, die Oligosaccharide und die Mucolipide umfasst, welche wenigstens eine Sialinsäuregruppe enthalten.

12. Reagenz gemäß einem der Ansprüche 10 und 11, dadurch gekennzeichnet, daß genanntes Substrat kovalent gekuppelt ist auf einem vorher durch Diazotierung mit Hilfe von $NaNO_2$ in saurer Lösung aktivierten festen Träger.

13. Reagenz gemäß einem der Ansprüche 10 bis 12, dadurch gekennzeichnet, daß es als Konservierungsmittel einen Glycin-Puffer, NaCl + 0.01% Na-Azid bei +4°C enthält.

14. Verfahren zur Herstellung des Reagenz zur Durchführung des Verfahrens gemäß Ansprüchen 7 und 8, dadurch gekennzeichnet, daß es die folgenden Stufen umfasst:

a) Behandlung der Säure-Latexkugeln mit während des Verfahrens aufgelöstem Mexamethylendiamin (HMD) in Gegenwart von 1-Ethyl-3,3-dimethylaminopropylcarbodimid (EDC), das in fester Form in drei Portionen in einem Zeitraum von 24 Stunden zugegeben wird,

b) Succinylierung der $NH_2$-Gruppen durch Zugabe Bernsteinsäureanhydrid bei einem pH größer 7,

c) Behandlung mit Hydrazinhydrochlorid in Anwesenheit von EDC,

d) kovalente Kupplung des Substrates der Neuraminidase, das mindestens eine Sialinsäuregruppe, vorzugsweise am Ende der Kette, enthält, auf den Säurehydrazid-Latexkugeln, die auf der vorherigen Stufe erhalten wurden, in einem entsprechenden Puffer.

15. Verfahren gemäß einem Anspruch 14, dadurch gekennzeichnet, daß der kovalenten Kupplung

des genannten Substrates auf den Säurehydrazid-Latexkugeln eine Aktivierung der genannten Kugeln durch Diazotierung mit Hilfe von $NaNO_2$ in saurer Lösung vorausgeht.

16. Verwendung des Reagenz gemäß einem der Ansprüche 10 bis 13 bei der Bestimmung von Anti-Neuraminidase-Antikörpern im Serum.

17. Diagnostischer Kit, dadurch gekennzeichnet, daß er als Reagenz ein Reagenz gemäß einem der Ansprüche 10 bis 13 oder ein Reagenz, hergestellt nach dem Verfahren des Anspruch 14, enthält.